# EUROPEAN PATENT SPECIFICATION

(11) **EP 4 275 723 B1**
(45) Date of publication and mention of the grant of the patent: **27.05.2026**
(21) Application number: 23172296.8
(22) Date of filing: 09.05.2023
(51) Int. Cl.: A61M 5/32, A61B 5/00, A61M 5/158

(54) **INSTRUMENT FOR A MEDICAMENT DELIVERY DEVICE THAT ACTS AS NEEDLE AND CANNULA**
INSTRUMENT FÜR EINE ALS NADEL UND KANÜLE WIRKENDE ARZNEIMITTELABGABEVORRICHTUNG
INSTRUMENT POUR UN DISPOSITIF D'ADMINISTRATION DE MÉDICAMENT QUI AGIT COMME UNE AIGUILLE ET UNE CANULE

(30) Priority: 10.05.2022 US 202263340063 P
(43) Date of publication of application: 15.11.2023
(73) Proprietor: Insulet Corporation, Acton, MA 01720 (US)
(72) Inventor: ZHAO, Jianhong, Carlisle (US); NAZZARO, David, Groveland (US); MCGAHREN, Lucas, Boston (US); DUHAMEL, Eric, Boxborough (US)
(74) Representative: Peterreins Schley

(56) References cited:
- WO-A1-02/28458
- WO-A2-02/081013
- DE-A1- 10 306 013
- US-A1- 2002 198 440
- US-A1- 2008 228 144
- US-A1- 2017 292 502

## Description

### BACKGROUND

U.S. Patent No. 10,898,696 discloses a drug delivery device that uses a combination of a cannula and a needle to facilitate drug delivery to a patient. Figure 1 depicts a high level diagram of components disclosed in U.S. Patent No. 10, 898,696 of a needle module 100 that effects the activation of the needle 104 and cannula 102 and retraction of the needle 104. The needle 104 is made of metal and is positioned inside the cylindrical inner lumen of the cannula 102. The cannula 102 is made of a softer material than the needle 104 and is more pliable. The difference between the exterior diameter of the needle 104 and the inner diameter of the cannula 102 is small but large enough to enable the needle 104 to be moved longitudinally in the inner lumen of the cannula 102. The cannula 102 may be formed of a pliable material, such as polytetrafluoroethylene (PTFE) that softens when exposed to body fluids and/or heat.

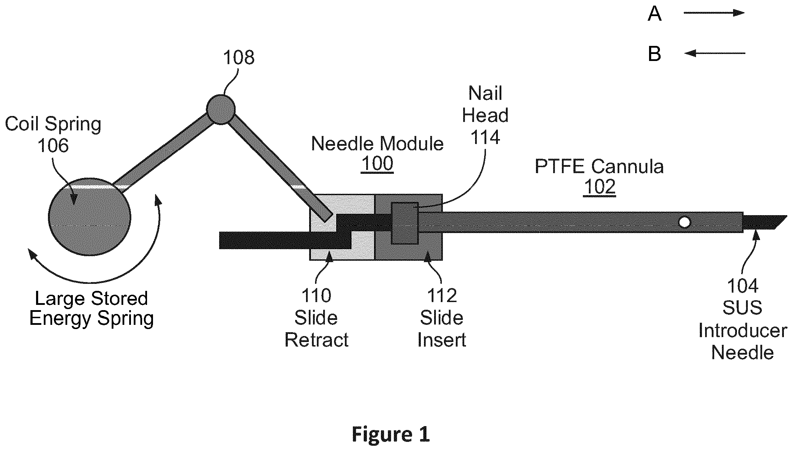

The needle 104 is coupled to a slide retract component 110 and passes through a slide insert component 112. The slide insert component 112 may include a pocket for holding a nail head 114 of the cannula 102. The slide insert component 112 may be coupled to the slide retract component 110 before the needle 104 pierces the skin of the patient. The slide insert component 112 and the slide retract component 110 may slide along a rail towards a stop (not shown) in direction A (see Figure 1). The slide insert component 112 is biased by the spring 106 to move toward the hard stop but is prevented from moving by a lock. When unlocked, the slide insert component 112 and the slide retract component 110 slide in tandem along the rail in direction A toward the hard stop responsive to the spring 106 pushing the linkage 108. This causes the needle 104 and cannula 102 to move toward the skin of the patient. Ultimately, the needle 104 pierces the skin of the patient and positions the cannula under the skin of the patient for delivery of a drug to the patient. The needle 104 is retracted when the slide retract decouples from the slide insert and is moved back in direction B.

There are several difficulties with the arrangement of Figure 1. First, it is difficult assemble. It is very labor intensive to put the needle 104 in cannula 102. Second, it is difficult to maintain a seal between the needle 104 and the cannula 102. Third, the arrangement occupies a good bit of space in the drug delivery device and prevents the drug delivery device from being smaller. The larger volume of the arrangement causes higher insertion angles for the needle and requires a bend in the needle. DE 103 06 013 A1 and US 2002/198440 A1 disclose medical instruments for piercing an injection site and delivering medicament, having characteristics that change when exposed to bodily fluids or temperature.

### SUMMARY

The invention is defined according to independent claims 1 and 5 with the dependent claims setting out further embodiments. In accordance with an inventive facet, an instrument for piercing the skin of a user and carrying a medicament to the user includes a cylindrical body having an inner lumen for carrying the medicament. The instrument further includes a piercing element secured to or formed on a distal end of the cylindrical body to pierce the skin of the user and to position the instrument at least partially under the skin of the patient. At least one of the cylindrical body or the piercing element is configured to soften upon the instrument being positioned at least partially under the skin of the user.

The cylindrical body is formed of a material that softens when the material is under the skin of the user. The material may be polytetrafluoroethylene (PTFE), high density polyethylene (HDPE), a polysaccharide, a polyhydroxyalkanoate (PHA), or polylactic acid (PLA). The cylindrical body may be coated with a dissolvable or degradable coating that hardens the cylindrical body and that dissolves or degrades on a portion of the cylindrical body that is under the skin of the user. The piercing element may include at least a portion that is hardened. The piercing element (in particular at least the portion of the piercing element referred to above) may be coated with a hardening coating that is configured to dissolve or degrade (including breaking away) upon being positioned under the skin of the user. The piercing element has a point or a sharpened edge. The instrument includes activatable elements that may be activatable to make at least a portion of the instrument rigid to facilitate piercing of the skin of the user. The activatable elements include shape memory alloy components that become rigid when electricity is applied to the shape memory alloy components.

In accordance with another inventive facet, a medicament delivery device includes a reservoir for storing medicament and an instrument for piercing skin of a user of the medicament delivery device and for remaining at least partially positioned under the skin of the user for carrying the medicament to the user. The instrument includes a cylindrical body having an inner lumen for carrying the medicament and a piercing element secured to or formed on an end of the cylindrical body to pierce the skin of the user and position the instrument at least partially under the skin of the patient. The cylindrical body is configured to soften upon the instrument being positioned at least partially under the skin of the user. A fluid path connects the reservoir with the instrument so that the medicament may pass from the reservoir to the instrument.

The cylindrical body of the instrument is formed of a material that softens when the material is under the skin of the user. The material may be PTFE. The cylindrical body of the instrument may be coated with a dissolvable or degradable coating that hardens the cylindrical body and that is configured to dissolve or degrade on a portion of the cylindrical body where that portion is under the skin of the user. The dissolvable or degradable coating may comprise or consist of a dissolvable or degradable polymer, such as polyvinyl alcohol (PVA), a starch-based polymer or a biodegradable polymer, like polylactic-co-glycolic acid (PLGA) or polyglycolic acid (PGA). The piercing element of the instrument may include at least a portion that is hardened. The piercing element (in particular at least the portion of the piercing element referred to above) of the instrument may be coated with a hardening coating that is configured to dissolve or degrade upon being positioned under the skin of the user. The piercing element of the instrument has a point or a sharpened edge. The medicament delivery device may be configured for delivering insulin to the user. The instrument includes activatable elements that may be activatable to make at least a portion of the instrument rigid to facilitate piercing of the skin of the user. The activatable elements include shape memory alloy components that become rigid when electricity is applied to the shape memory alloy components.

In accordance with a further inventive facet, an insulin delivery device includes a reservoir for storing insulin and a needle or cannula for piercing skin of a user of the insulin delivery device and for remaining at least partially positioned under the skin of the user for carrying the insulin to the user. The needle or cannula includes a cylindrical body having an inner lumen for carrying the insulin and a piercing element secured to or formed on an end of the cylindrical body to pierce the skin of the user and position the instrument at least partially under the skin of the patient. The insulin delivery device further includes a fluid path connecting the reservoir with the instrument so that the insulin may pass from the reservoir to the needle or cannula.

### BRIEF DESCRIPTION OF THE DRAWINGS

Figure 1 depicts a conventional arrangement with a cannula and needle used by a conventional drug delivery device.
Figure 2 depicts a block diagram of a medicament delivery device of exemplary embodiments.
Figure 3A depicts an illustrative instrument of exemplary embodiments that has a pointed tip formed by a dissolvable or degradable coating.
Figure 3B depicts a cross-sectional view of the body of the instrument of Figure 3A.
Figure 3C depicts a flowchart of illustrative steps that may be performed in exemplary embodiments to form the instrument of Figure 3A.
Figure 3D depicts a flowchart of illustrative steps that may be performed in exemplary embodiments to use the instrument of Figure 3A.
Figure 4 depicts an arrangement of the actuator and instrument in exemplary embodiments.
Figure 5A depicts an instrument of exemplary embodiments that includes a dissolvable or degradable coating on the cylindrical body of the instrument where the coating forms a pointed tip for piercing.
Figure 5B depicts a flowchart of illustrative steps that may be performed in exemplary embodiments to form the instrument of Figure 5A.
Figure 5C depicts a flowchart of illustrative steps that may be performed in exemplary embodiments to use the instrument of Figure 5A.
Figure 6A depicts an illustrative instrument of exemplary embodiments that includes a hardened insert at the distal end for piercing.
Figure 6B depicts an illustrative instrument of exemplary embodiments that includes a hardened cap at the distal end for piercing.
Figure 6C depicts a flowchart of illustrative steps that may be performed in exemplary embodiments to form the instruments of Figures 6A and 6B.
Figure 6D depicts a flowchart of illustrative steps that may be performed in exemplary embodiments to use the instruments of Figures 6A and 6B.
Figure 7 depicts a PTFE cannula with a stiff tip of an exemplary embodiment positioned beneath the skin of a patient.
Figure 8A depicts an instrument of the exemplary embodiments formed of a singular material and having a pointed tip for piercing.
Figure 8B depicts a flowchart of illustrative steps that may be performed in exemplary embodiments to form the instrument of Figure 8A.
Figure 8C depicts a flowchart of illustrative steps that may be performed in exemplary embodiments to use the instrument of Figure 8A.
Figure 9A depicts a first alternative for an instrument that has activatable elements that may become in rigid in exemplary embodiments.
Figure 9B depicts a second alternative for an instrument that has activatable elements that may become in rigid in exemplary embodiments.
Figure 10 depicts a flowchart of illustrative steps that may be performed in exemplary embodiments to form the instruments of Figures 9A and 9B.
Figure 11 depicts a flowchart of illustrative steps that may be performed in exemplary embodiments to use the instruments of Figures 9A and 9B.

### DETAILED DESCRIPTION

Exemplary embodiments may provide a medicament delivery device that includes a single instrument for piercing the skin of the user and for acting as a conduit for delivering medicament to the user subcutaneously. There is no need for both a needle for piercing the skin of the user and a cannula for serving as a conduit for delivering the medicament subcutaneously to the user. The use of the single instrument in exemplary embodiments reduces the complexity, labor needs for assembly, and cost of assembly. The instruments of the exemplary embodiments also eliminate challenges faced by conventional approaches, such as providing a good seal between the needle and cannula as described above. Moreover, the instrument assembly for inserting and retracting the instrument may occupy less volume than found with conventional medicament delivery devices.

The exemplary embodiments may reduce discomfort caused by a rigid cannula or needle. The exemplary embodiments may provide instruments that are configured to be comfortable to the user when the instruments are in place beneath the skin of the user, such as when a medicament delivery device is delivering medicament to the user.

The instrument includes a cylindrical body that contains an inner lumen. The inner lumen may serve as a conduit for delivering medicament from a reservoir to the user after the instrument is positioned subcutaneously. The cylindrical body may be formed of a material that normally is rigid but which softens to become semi-rigid but more pliable when positioned subcutaneously due to exposure to body fluids and/or heat. The softening of the material may increase the comfort to the user when the instrument is positioned subcutaneously. In some exemplary embodiments, the cylindrical body may have a hardening coating on its outer surface to make the instrument more rigid. The coating may dissolve or degrade when subject to bodily fluids and/or warmth (e.g. heat), such as for the coating on the portion of the cylindrical body that is positioned beneath the skin of the patient. That portion of the cylindrical body beneath the skin of the user may soften and become more pliable. The term "softening" relates to a reduction of mechanical strength, e.g. Young's Modulus, of a material, body, or structure compared to a state prior to the "softening". The term "hardening" relates to an increase of mechanical strength, e.g. Young's Modulus, of a material, body, or structure compared to a state prior to the "hardening"

The distal end of the instrument may contain a piercing element. The piercing element may be a pointed or sharpened end. In some exemplary embodiments, the piercing element may be formed by a coating applied to the distal end of the cylindrical body. The coating may form a point for piercing the skin of the user. The coating may be designed to dissolve when positioned below the skin of the user. Alternatively, a hardened element may be positioned on the distal end of the cylindrical body to act as the piercing element. The hardened element may be made of metal, such as surgical stainless steel, and may be place over the distal end like a cap, inlaid into a tip or even formed integrally with the cylindrical body.

In some exemplary embodiments, the instrument may include activatable elements that may make the instrument more rigid when activated. For example, shape memory alloys may be positioned on the outer surface of the instrument. When a sufficient voltage (or electricity) is applied to the shape memory alloy, the shape memory alloy becomes rigid and makes the instrument rigid. When the voltage is no longer applied or sufficiently reduced, the shape memory alloy and correspondingly the instrument may become less rigid. Thus, the instrument may be made rigid during piercing of the skin of a user and less rigid when positioned subcutaneously.

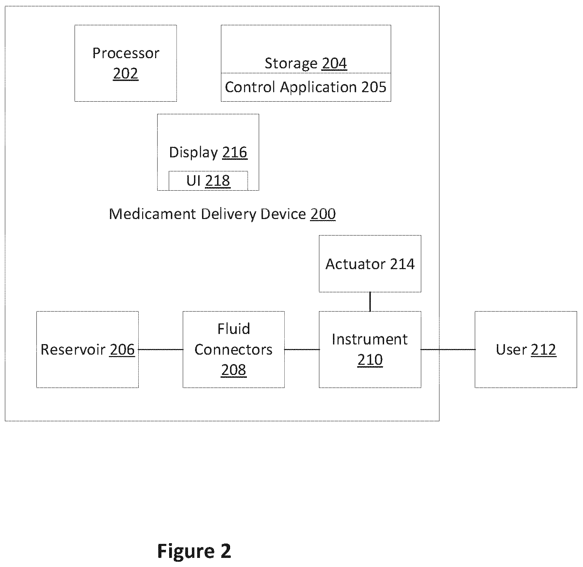

Figure 2 depicts a block diagram of an illustrative medicament delivery device 200 for exemplary embodiments. The medicament delivery device 200 may be an insulin pump in some exemplary embodiments or a device that delivers multiple medicaments. The medicament delivery device 200 delivers medicament in fluid form to a user 212. The medicament delivery device 200 may be directly coupled to a user (e.g., directly attached to a body part and/or skin of the user 212 via an adhesive or the like). The medicament is stored in fluid form (such as in liquid form) in a reservoir 206. The medicament delivery device 200 includes a processor 202. The processor 202 may be, for example, a microprocessor, a logic circuit, a field programmable gate array (FPGA), an application specific integrated circuit (ASIC) or a microcontroller. The processor 110 may maintain a date and time as well as other functions (e.g., calculations or the like). The processor 202 may execute a control application 205 stored in a storage 204 for controlling the operation of the medicament delivery device 200. The storage 204 may include both primary memory and secondary memory. The storage 204 may include random access memory (RAM), read only memory (ROM), optical storage, magnetic storage, removable storage media, solid state storage or the like. A display 216, such as an LCD display, an LED display or a touchscreen, may be provided for displaying a user interface 218.

The processor 202 running the control application 205 controls the dispensing of the medicament from the reservoir 206 through fluid connectors 208, like tubing and valves, to instrument 210 and ultimately to the user. In some embodiments, there may be no need for fluid connectors 208; rather there may be a direct connection between the reservoir 206 and the instrument 210. The instrument 210 is activated to pierce the skin of the user 212 and serves as a conduit to deliver the medicament to the user 212. The control application 205 contains computer programming instructions for controlling the medicament delivery device 200, including causing the instrument 210 to pierce the skin of the user 212 via actuator 214, positioning the instrument 210 subcutaneously via actuator 214 and causing delivery of the medicament. The piercing of the skin of the user 212 is part of the initialization of the medicament delivery device 200 once secured to the skin of the user 212. The actuator 214 may be a spring and rail assembly as described in more detail below.

A wide variety of medicaments may be delivered by the medicament delivery device 200. The medicament may be insulin for treating diabetes. The medicament may be glucagon for raising a user's glucose level. The medicament also may be a glucagon-like peptide (GLP)-1 receptor agonists for lowering glucose or slowing gastric emptying, thereby delaying spikes in glucose after a meal. Alternatively, the medicament delivered by the medicament delivery device 200 may be one or a combination of a pain relief agent, a chemotherapy agent, an antibiotic, a blood thinning agent, a hormone, a blood pressure lowering agent, an antidepressant, an antipsychotic, a statin, an anticoagulant, an anticonvulsant, an antihistamine, an antiinflammatory, a steroid, an immunosuppressive agent, an antianxiety agent, an antiviral agents, a nutritional supplement or a vitamin.

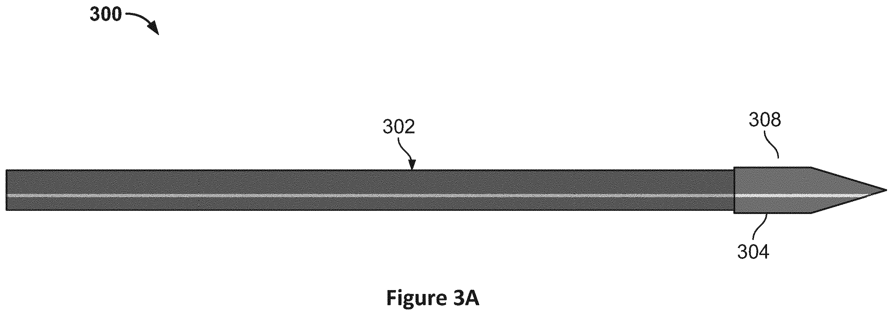

Figure 3A depicts an illustrative instrument 300 that may be used in exemplary embodiments. The instrument 300 may be used to pierce the skin of the user 212 and to serve as a conduit for subcutaneous delivery of the medicament from the reservoir 206 to the user 212. The instrument 300 has a cylindrical body 302 formed of a polymer, such as high density polyethylene (HDPE), a polysaccharide, a polyhydroxyalkanoate (PHA) or polylactic acid (PLA). These polymers are rigid prior to exposure to body fluids and/or body heat, whereupon they soften to semi-rigid state. A piercing element 304 is formed on the distal end 308 of the cylindrical body 302. The piercing element 304 may be a sleeve that fits over the distal end 308 of the cylindrical body 302 and is fused to the distal end 308. The piercing element 304 is formed as a pointed piercing tip formed of a dissolvable or degradable polymer, such as polyvinyl alcohol (PVA), a starch-based polymer or a biodegradable polymer, like polylactic-co-glycolic acid (PLGA) or polyglycolic acid (PGA). The piercing element 304 is configured to dissolve or degrade including breaking away from the instrument 300 when exposed to body fluids and heat after the piercing element is positioned underneath the skin of the user 212. The cylindrical body 302 that is not beneath the skin of the user 212 remains rigid, but the portion under the skin softens. Thus, the rigid cylindrical body 302 and pointed tip 304 of the piercing element are useful when piercing the skin of the user 212, but the portions under the skin of the user after piercing soften to enhance the comfort to the user 212.

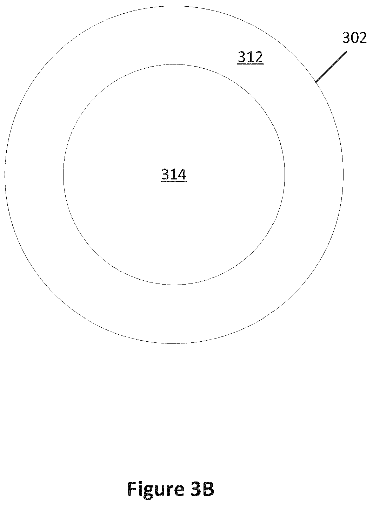

Figure 3B depicts a cross-sectional view of the cylindrical body 302. The cylindrical body contains an inner lumen 314 that serves as a conduit for carrying the medicament to the user 212. The depiction in Figure 3B shows a cylindrical inner lumen 314. Nevertheless, other profiles of the inner lumen may be used. For instance, the inner lumen could have an oval profile, a rectangular profile, etc. Similarly the surrounding concentric portion 312 of the cylindrical body 302 need not have a circular profile but may instead have an oval profile, a rectangular profile, etc.

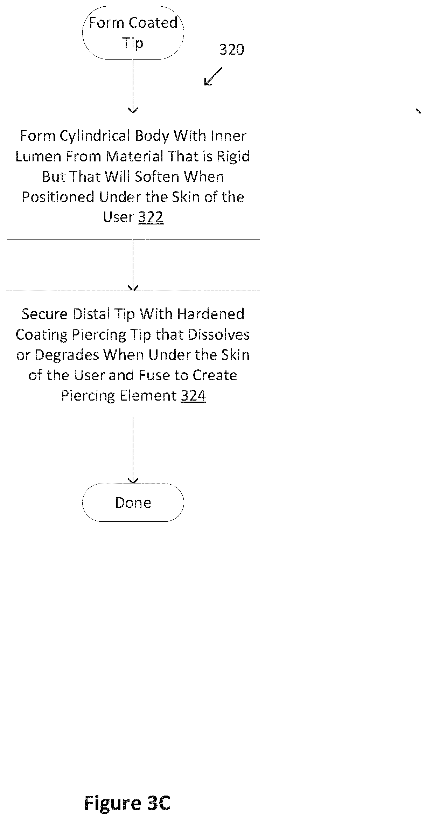

Figure 3C depicts a flowchart of illustrative steps that may be performed in exemplary embodiments to form the instrument 300. At 322, the cylindrical body 302 is formed of a material that is rigid but that will soften when positioned under the skin of the user 212. Examples of suitable materials have been discussed above. At 324, a distal tip with a hardened coating piercing tip 304 that dissolves or degrades when positioned under the skin of the user 212 is secured to the cylindrical body and fused to create a piercing element.

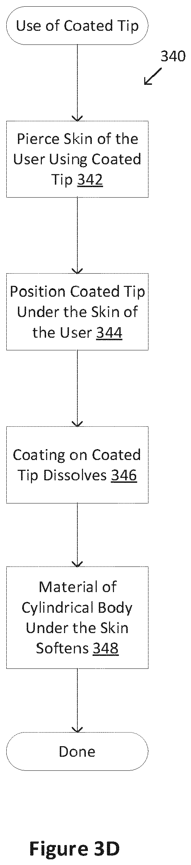

Figure 3D depicts a flowchart 240 of illustrative steps that may be performed in exemplary embodiments to use an instrument, like instrument 300 of Figure 3A. At 342, the skin of the user 212 is pierced using the coated tip 304. At 344, the coated tip 304 is positioned under the skin of the user 212. The piercing and positioning of 342 and 344 may be performed by mechanisms of the medicament delivery device as will be explained in more detail below. Given the exposure to body fluids and/or body heat under the skin of the user 212, at 346, the coated tip 304 dissolves or degrades. At least a portion of the cylindrical body 304 at the distal end 308 also may be exposed to the body fluids and heat as the portion is beneath the skin of the user 212. Thus, at 348, the material of the cylindrical body 302 under the skin softens. The dissolving or degrading of the coated tip 304 and the softening of the material in the cylindrical body 302 may occur rapidly, such as within a single minute or within a few minutes (such as 2 or 3 minutes). Alternatively, sometimes it may take longer, such as 30 minutes, for the softening of the material.

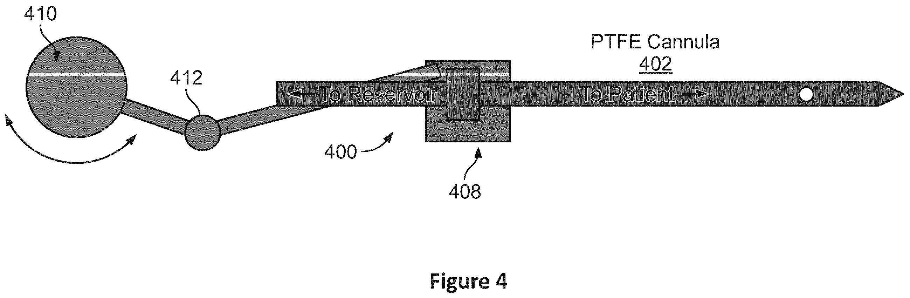

Figure 4 depicts a diagram of illustrative components 400 of the actuator 214 and instrument 210 for actuating and retracting the instrument 210 that may be found in exemplary embodiments. The components may be simplified due to the use of the single instrument 402 rather than both a needle and a cannula. A slide component 408 is provided to slide the instrument 402 toward the user 212 to pierce the skin of the user 212 and position the instrument under the skin of the user 212 and also to slide the instrument 402 away from the user toward the reservoir 206. The slide component 408 rides on a rail. The motion of the slide component 408 on the rail is driven by a spring 410 that via a linkage 412. A nail head 414 of the instrument 402 is held in a pocket of the slide component 408. The spring may move the slide component 408 toward the user 212 to pierce the skin of the user and position the instrument under the skin of the user 212 and also retract the instrument 402 as needed. Since there is not both a needle and a cannula, there is no need for a seal between the needle and cannula. Moreover, there is no need for a retract slide component. The arrangement of Figure 4 is more easily assembled than the conventional arrangement of Figure 1 and may occupy less volume than the arrangement of Figure 1.

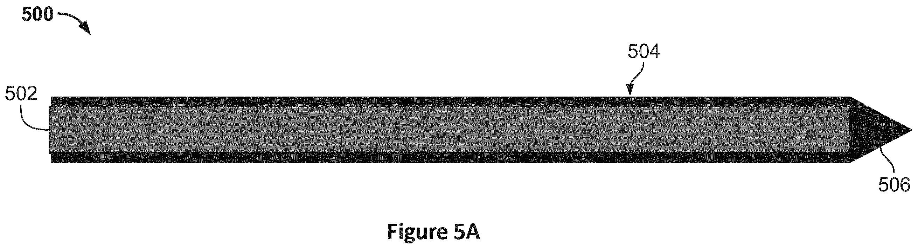

The instrument 210 is not limited to that shown in Figure 4A. Figure 5A depicts another example of an instrument 500 of the exemplary embodiments. The instrument 500 includes a cylindrical body 502 having an inner lumen for carrying medicament. The cylindrical body 502 may be formed of a suitable material, such as PTFE. The cylindrical body 502 is coated with a dissolvable or degradable coating 504 to make the cylindrical body 502 rigid and to form a piercing tip 506. The coating 504 dissolves or degrades when exposed to body fluid and body heat, such as when under the skin of the user 212. The coating forms a pointed end 506 that serves as a piercing element. The pointed end 506 dissolves or degrades when positioned under the skin of the user 212. The portion of the coating 504 on the cylindrical body 502 that is beneath the skin of the user 212 also dissolves or degrades. As a result, the comfort to the user 212 is enhanced and greater angles of insertion are permitted because the instrument is softened at the distal end.

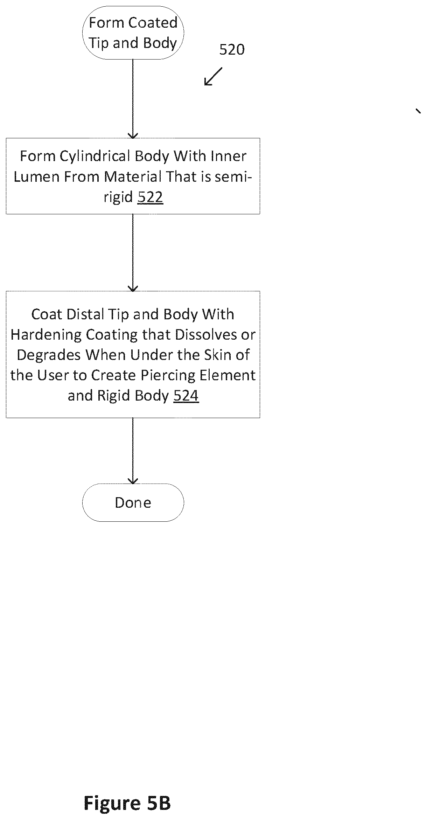

Figure 5B depicts a flowchart 520 of illustrative steps that may be performed in exemplary embodiments to form the instrument 500. At 522, the cylindrical body 502 with an inner lumen is formed from material that is semi-rigid, such as PTFE. The material may hold its shape but may be pliable. The cylindrical body may be formed, for instance, by extrusion or by molding. At 524, the distal tip and body of the cylindrical body 502 is coated with a hardening coating that dissolves or degrades when under the skin of the user 212. The hardening coating may comprise or consist of a dissolvable or degradable polymer, such as polyvinyl alcohol (PVA), a starch-based polymer or a biodegradable polymer, like polylactic-co-glycolic acid (PLGA) or polyglycolic acid (PGA). The coating 504 may form a piercing tip 506 that serves as a piercing element for piercing the skin of the user 212. Accordingly, in some embodiments the piercing element may comprise or consist of a dissolvable or degradable polymer, such as polyvinyl alcohol (PVA), a starch-based polymer or a biodegradable polymer, like polylactic-co-glycolic acid (PLGA) or polyglycolic acid (PGA). The term "hardening coating" relates to a coating that increases the mechanical strength, e.g. Young's Modulus, of a material, body, or structure (prior to dissolving or softening).

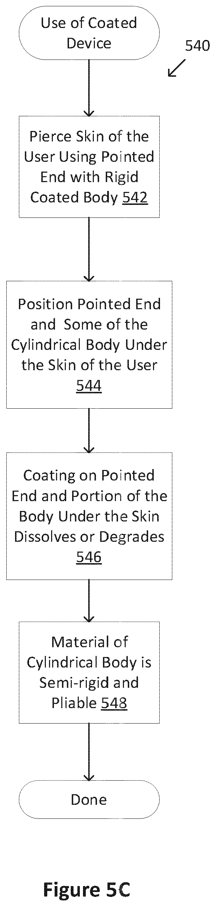

Figure 5C depicts a flowchart 540 of illustrative steps that may be performed in exemplary embodiments in using the instrument 500. Initially, at 542, the skin of the user is pierced using the pointed end 506 with the rigid coated cylindrical body (see 502 and 504). At 544, the pointed end 506 and a distal portion of the cylindrical body 502 are positioned under the skin of the user 212. The piercing of the skin at 542 and the positioning of the pointed end 506 under the skin of the user 212 are performed by the actuator 214 under the control of the control application 205. A user interface may be displayed on display 216 to provide an element that may be selected by the user to cause deployment of the instrument 210 to pierce the skin and position at least a portion of the instrument 210 under the skin of the user 212. At 546, the coating on the pointed end 506 and the portion of the cylindrical body 502 under the skin of the user 212 dissolves or degrades. At 548, the material of the cylindrical body without the coating is semi-rigid and pliable. Thus, the instrument 500 may have greater range of angles of insertion.

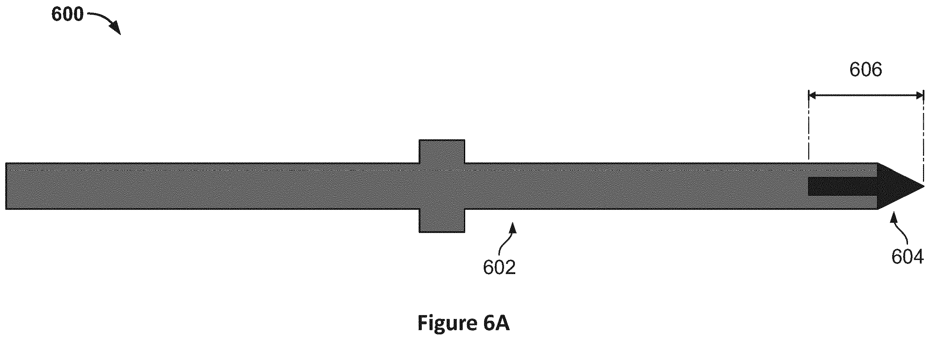

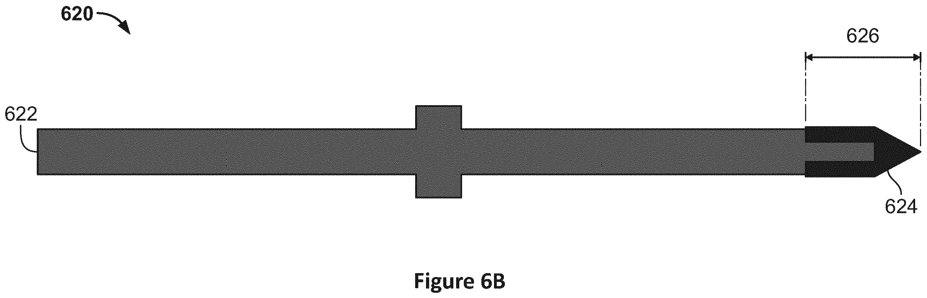

Figure 6A depicts another exemplary embodiments of an instrument 600. The instrument 600 has a cylindrical body 602 formed of a semi-rigid material, such as PTFE. The cylindrical body 600 has an inner lumen, such as described above, for carrying medicament. A pointed tip 606 is formed by an element of stiff material, such as metal. The pointed tip 604 acts as a piercing element for piercing the skin of the user 212. The element may be press fit into distal end of the cylindrical body 602. Reference 606 identifies the length of the element of stiff material of the pointed tip 604. Figure 6B shows another alternative for the pointed tip 624 of length 626 that fits over the distal end of the cylindrical body 622 of the instrument 620. The pointed tip 624 is formed of a stiff material, such as a metal. It such be appreciated that other configurations and designs may be used for the pointed tip. Those shown in Figures 6A and 6B are intended to be illustrative and not limiting.

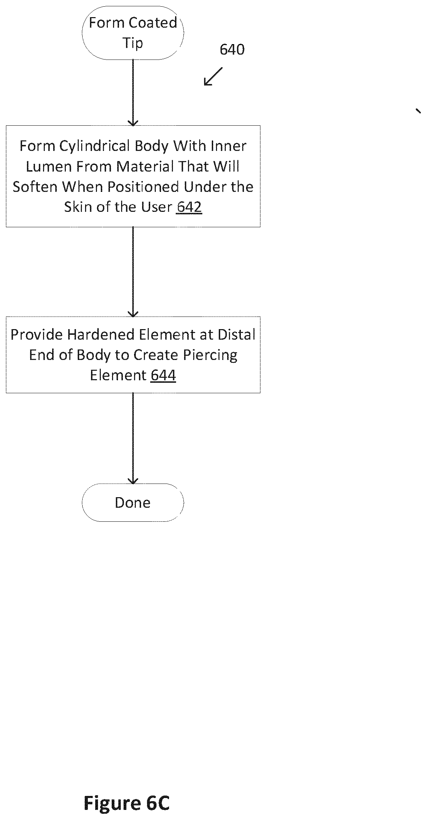

Figure 6C depicts a flowchart 640 of illustrative steps that may be performed in exemplary embodiments to form the instruments 600 or 620. At 642, the cylindrical body 602, 622 is formed from a semi-rigid material like PTFE that will soften when positioned under the skin of the user 212. The cylindrical body 602, 622 may be formed by extrusion or molding, for instance. At 644, the hardened element of stiff material 604, 624 is provided at the end of the cylindrical body 602, 622 to form a piercing element. The hardened element 604, 624 may be in the form of a sleeve that is secured to the cylindrical body 602, 622. In other instances, the hardened element 604, 624 may be an insert that is press fit into the distal end of the cylindrical body 602, 622.

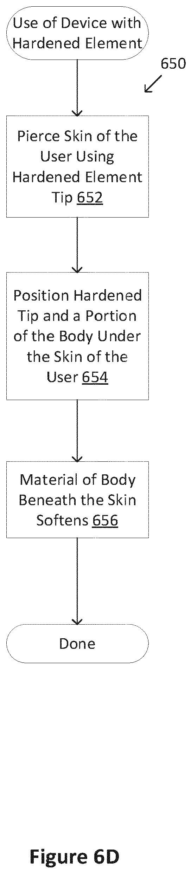

Figure 6D depicts a flowchart 650 of illustrative steps that may be performed in exemplary embodiments to use the instruments 600 or 620. At 652, the hardened element tip 604, 624 is used to pierce the skin of the user 212. The hardened tip 604, 624 and a portion of the cylindrical body 602, 622 are positioned under the skin of the user 212. At 656, the material of the cylindrical body 602, 622 that is below the skin of the user softens and becomes more pliable. Thus, the comfort of the user is enhanced and more severe insertion angles are possible.

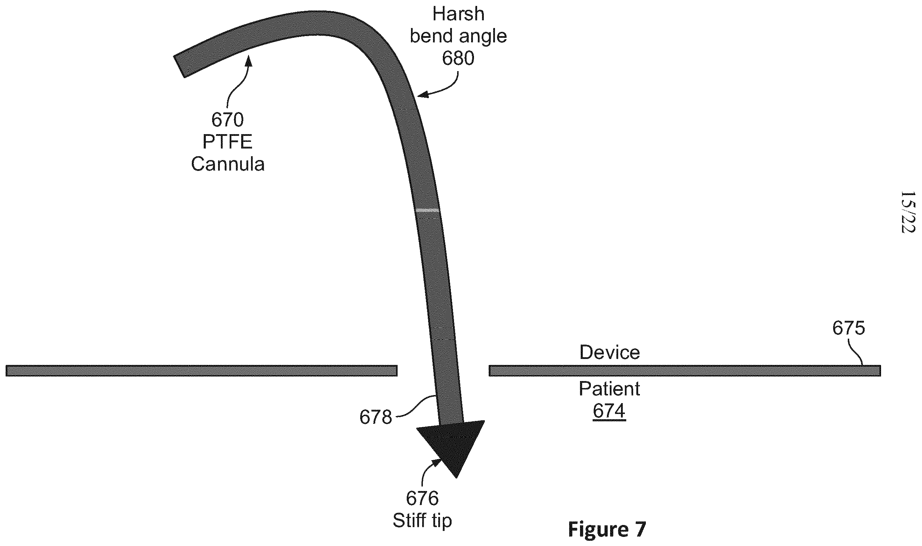

Figure 7 depicts an example of a PTFE cannula 670 (such as those depicted in Figures 6A and 6B) with a stiff tip 676 positioned subcutaneously under the skin 675 of a patient 674. This positioning may be found after activation of the cannula so that the skin 675 of the patient 674 has been pierced by the stiff tip 676 and the leading distal portion 678 of PTFE cannula 670 is in under the skin 675. The body fluids and/or body heat of the patient 674 may soften the PTFE cannula 670. As a result, the PTFE cannula 670 may bend to assume a harsh bend angle 680 as shown in Figure 7.

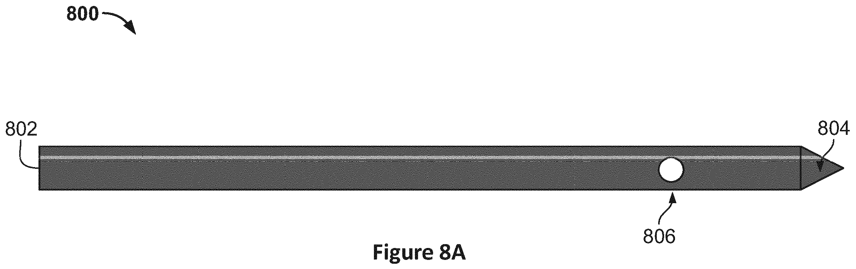

In some exemplary embodiments, the instrument and piercing tip may be formed of the same material and may be integrally formed. Figure 8A depicts an instrument 800 formed of a rigid material that softens when exposed to body fluids and/or body heat. Examples of suitable materials include but are not limited to HDPE, a polysaccharide, PHA or PLA. The instrument 800 includes a cylindrical body 802 with an inner lumen for carrying medicament. The instrument 800 includes a pointed tip 804 that serves as a piercing element. The pointed tip 804 is formed of the same material as the cylindrical body. In some alternative embodiments, the piercing element 804 is formed of another type of polymer that softens when exposed to body fluids and/or body heat. The cylindrical body 802 includes a port 806 for outputting the medicament from the inner lumen to the user 212.

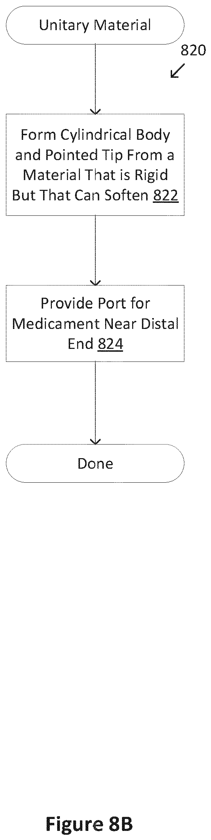

Figure 8B depicts a flowchart 820 of illustrative steps that may be performed in exemplary embodiments to form an instrument, like instrument 800. At 822, the cylindrical body 802 and the pointed tip 804 are formed from a material that is rigid but that can soften. The formation may entail extrusion and/or molding. At 824, a port 806 may be formed near the distal end of the cylindrical body 802. The port 806 may be formed by drilling, molding or by other techniques.

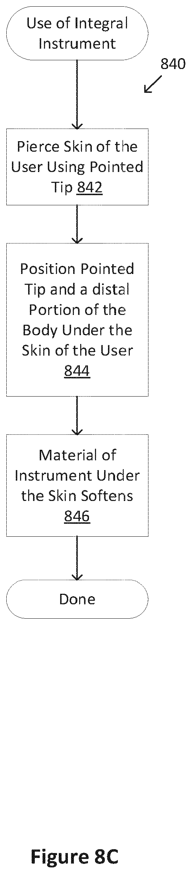

Figure 8C depicts a flowchart 840 of illustrative steps that may be performed in exemplary embodiments to use the integral instrument 800. At 842, the pointed tip 804 is used to pierce the skin of the user 212. At 844, the pointed tip 804 is positioned under the skin of the suer 212 along with a distal portion of the cylindrical body 802. At 846, the material of the pointed tip 804 and of the distal portion of the cylindrical body 802 under the skin of the user 212 softens. This enhances the comfort of the user 212 and facilitates more acute insertion angles.

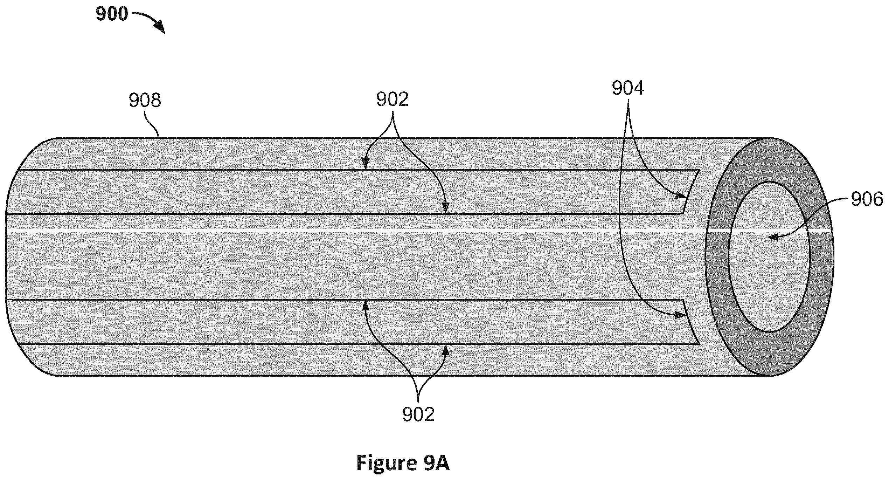

The instrument may also be configured to have activatable elements that may be activated to change the stiffness of the instrument. Figure 9A depicts an illustrative example of an instrument 900 that contains activatable elements that may be activated to cause the instrument to become more rigid. The activatable elements take the form of shape memory alloy (SMA) wires 902 organized in pairs., The pairs of SMA wires 902 are distributed evenly around the circumference and length of the exterior of the instrument 900. The cylindrical body 908 of the instrument 900 is formed of a soft material. The SMA wires 902 are embedded in the soft material or molded into the soft material. An electrical shunt 904 may be provided for each pair of SMA wires. The shape memory alloy wires 902 stiffen upon the application of a sufficient voltage to the SMA wires 902. The electrical shunts 904 may be made of non-shape memory alloy so that they do not stiffen upon application of electricity to the wires 902. The cylindrical body 908 includes an inner lumen 906 for carrying medicament.

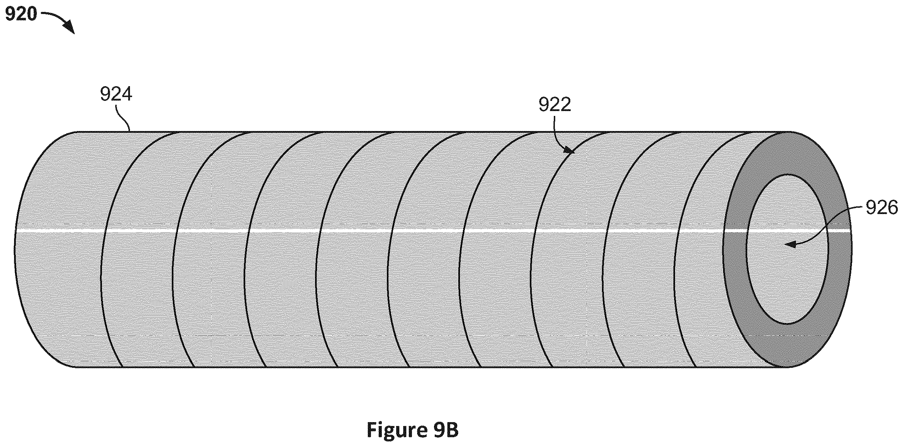

Figure 9B depicts another instrument 920 that has activatable elements. In this instrument 920, A SMA wire 922 is helically wrapped over the length of the cylindrical body 924. The cylindrical body 924 is made of a soft material. When electricity is applied (i.e., a sufficient voltage), the SMA wire 922 stiffens and thus stiffens the instrument 920. The cylindrical body 924 includes an inner lumen for carrying medicament for delivery to the user 212.

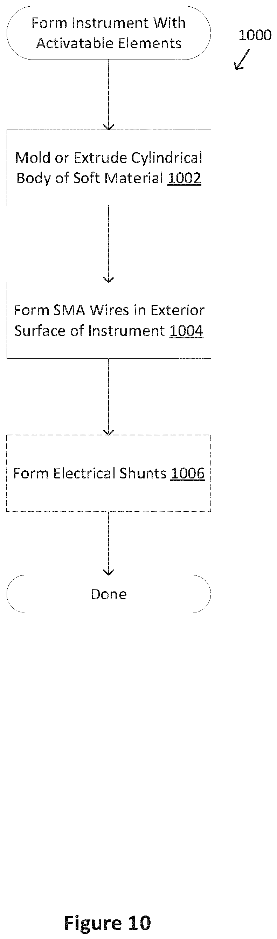

Figure 10 depicts a flowchart of illustrative steps that may be performed to form the instruments 900, 920 of Figures 9A and 9B in exemplary embodiments. At 1002 the cylindrical body 908, 924 is formed of a soft material, such as by extrusion or molding. At 1004, the SMA wire(s) 902, 922 are formed on the exterior surface of the instrument 900, 920. The SMA wire(s) 902, 922 extend around the circumference of the instrument 900, 920 and along the length of the instrument 900, 920. The SMA wire(s) 902, 922 may be embedded in or molded in the material of the cylindrical body 908, 924. Optionally, at 1006, electrical shunts 904 may be formed if warranted, depending on the configuration of the SMA wire(s) 902, 922.

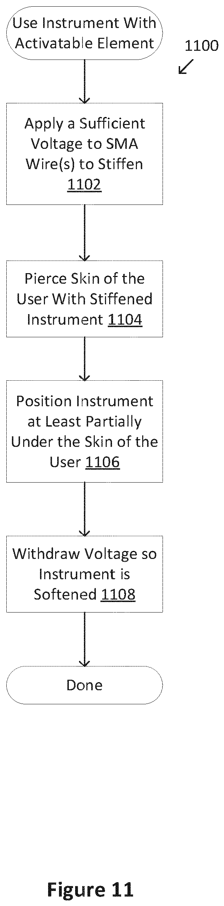

Figure 11 depicts a flowchart 1100 of illustrative steps that may be performed in exemplary embodiments in using the instruments 900, 920 of Figures 9A and 9B. At 1102, a sufficient voltage is applied to the SMA wire(s) 902, 922 to stiffen the wires and the instrument 900, 920. At 1104, the skin of the user 212 is pierced with the stiffened instrument 902, 922. Tat 1106, the instrument 900, 920 is positioned at least partially under the skin of the user 212 (e.g., a distal end is under the skin). Once the instrument is properly positioned under the skin of the user 212, at 1108, the voltage is withdrawn from the SMA wire(s) 902, 922 to soften the wires and instrument.

While exemplary embodiments have been described herein, various changes in form and detail may be made without departing from the intended scope of the claims appended hereto.

## Claims

1. An instrument for piercing skin of a user and carrying a medicament to the user, comprising:
a cylindrical body having an inner lumen for carrying the medicament;
a piercing element formed as a pointed piercing tip of dissolvable or degradable polymer and being secured to a distal end of the cylindrical body to pierce the skin of the user and position the instrument at least partially under the skin of the user; and
wherein the cylindrical body is configured to soften, and the piercing element is configured to dissolve or degrade including breaking away from the instrument (300) upon being positioned at least partially under the skin of the user,
**characterized in that** the cylindrical body is formed of a material that is rigid prior to exposure to body fluids and/or body heat but that softens when the material is under the skin of the user.

2. The instrument of claim 1, wherein the material of the cylindrical body is polytetrafluoroethylene (PTFE), high density polyethylene (HDPE), a polysaccharide, a polyhydroxyalkanoate (PHA), or a polylactic acid.

3. The instrument of claim 1 or 2, wherein the material of the cylindrical body softens to a semi-rigid state when being positioned under the skin of a user.

4. The instrument of any one of claims 2 to 3, wherein the material of the piercing element comprises or consists of a dissolvable or degradable polymer, such as polyvinyl alcohol (PVA), a starch-based polymer or a biodegradable polymer, like polylactic-co-glycolic acid (PLGA) or polyglycolic acid (PGA).

5. An instrument for piercing skin of a user and carrying a medicament to the user, comprising:
a cylindrical body formed of a soft material and having an inner lumen for carrying the medicament;
activatable elements that are embedded or molded into the soft material and **characterized in that** the activatable elements are activatable to cause the instrument to become more rigid to facilitate piercing of the skin of the user,
wherein the activatable elements are organized in pairs distributed evenly around the circumference and length of the exterior of the instrument,
wherein the activatable elements take the form of shape memory alloy (SMA) wires that stiffen when electricity is applied to the SMA wires, and thus stiffen the instrument.

6. The instrument of claim 5, wherein the SMA wires are helically wrapped over a length of the cylindrical body.

7. A medicament delivery device, comprising:
a reservoir for storing a medicament;
the instrument according to any one of the preceding claims, wherein the instrument is configured for remaining at least partially positioned under the skin of the user for carrying the medicament to the user; and
a fluid path connecting the reservoir with the instrument so that the medicament may pass from the reservoir to the instrument.

8. The medicament delivery device of claim 7, further comprising:
a slide component for sliding the instrument to a first position for piercing the skin of the user and positioning the instrument under the skin of the user and for sliding the instrument away from the user into a second position where the instrument is retracted from the user; and a rail, a spring and a linkage connected to the spring and the slide component for driving the single slide component.

9. The medicament delivery device of any one of claims 7 to 8, wherein the medicament delivery device is an insulin delivery device configured for delivering insulin to the user.

## Patentansprüche

1. Instrument zum Durchstechen der Haut eines Benutzers und Befördern eines Medikaments zu dem Benutzer, umfassend:
einen zylindrischen Körper mit einem inneren Lumen zum Befördern des Medikaments;
ein Stechelement, das als spitze Stechspitze aus löslichem oder abbaubarem Polymer ausgebildet ist und an einem distalen Ende des zylindrischen Körpers angebracht ist, um die Haut des Benutzers zu durchstechen und das Instrument wenigstens zum Teil unter der Haut des Benutzers zu positionieren; und
wobei der zylindrische Körper dafür gestaltet ist, weich zu werden, und das Stechelement dafür gestaltet ist, sich aufzulösen oder abzubauen, einschließlich Abbrechen von dem Instrument (300), nachdem es wenigstens zum Teil unter der Haut des Benutzers positioniert ist,
**dadurch gekennzeichnet, dass**
der zylindrische Körper aus einem Material gebildet ist, das vor Exposition gegenüber Körperfluiden und/oder Körperwärme starr ist, aber weich wird, wenn sich das Material unter der Haut des Benutzers befindet.

2. Instrument nach Anspruch 1, wobei das Material des zylindrischen Körpers Polytetrafluorethylen (PTFE), Polyethylen hoher Dichte (HDPE), ein Polysaccharid, ein Polyhydroxyalkanoat (PHA) oder eine Polymilchsäure ist.

3. Instrument nach Anspruch 1 oder 2, wobei das Material des zylindrischen Körpers zu einem halbstarren Zustand weich wird, wenn es unter der Haut eines Benutzers positioniert wird.

4. Instrument nach einem der Ansprüche 2 bis 3, wobei das Material des Stechelements ein lösliches oder abbaubares Polymer, wie z.B. Polyvinylalkohol (PVA), ein Polymer auf Stärkebasis oder ein biologisch abbaubares Polymer, wie z.B. Polymilchsäure-co-glycolsäure (PLGA) oder Polyglycolsäure (PGA), umfasst oder daraus besteht.

5. Instrument zum Durchstechen der Haut eines Benutzers und Befördern eines Medikaments zu dem Benutzer, umfassend:
einen zylindrischen Körper, der aus einem weichen Material ausgbeildet ist und ein inneres Lumen zum Befördern des Medikaments aufweist;
aktivierbare Elemente, die in das weiche Material eingebettet oder eingeformt sind, **dadurch gekennzeichnet, dass** die aktivierbaren Elemente aktivierbar sind, um zu bewirken, dass das Instrument steifer wird, um Durchstechen der Haut des Benutzers zu erleichtern,
wobei die aktivierbaren Elemente in Paaren organisiert sind, die gleichmäßig um den Umfang und die Länge der Außenseite des Instruments verteilt sind,
wobei die aktivierbaren Elemente die Form von Drähten aus Formgedächtnislegierung (SMA) annehmen, die versteifen, wenn Elektrizität an die SMA-Drähte angelegt wird, und damit das Instrument versteifen.

6. Instrument nach Anspruch 5, wobei der eine oder die mehreren SMA-Drähte schraubenförmig über eine Länge des zylindrischen Körpers gewickelt sind.

7. Medikamentenabgabevorrichtung umfassend:
ein Reservoir zum Aufbewahren eines Medikaments;
das Instrument nach einem der vorstehenden Ansprüche, wobei das Instrument dafür gestaltet ist, wenigstens zum Teil unter der Haut des Benutzers positioniert zu bleiben, um das Medikament zu dem Benutzer zu befördern; und
einen Fluidweg, der das Reservoir mit dem Instrument verbindet, so dass das Medikament aus dem Reservoir zu dem Instrument gelangen kann.

8. Medikamentenabgabevorrichtung nach Anspruch 7, ferner umfassend:
eine Schiebekomponente zum Schieben des Instruments in eine erste Position zum Durchstechen der Haut des Benutzers und Positionieren des Instruments unter der Haut des Benutzers, und zum Schieben des Instruments von dem Benutzer weg in eine zweite Position, in der das Instrument von dem Benutzer zurückgezogen ist;
und eine Schiene, eine Feder und ein mit der Feder und der Schiebekomponente verbundenes Verbindungselement zum Antreiben der einzelnen Schiebekomponente.

9. Medikamentenabgabevorrichtung nach einem der Ansprüche 7 bis 8, wobei die Medikamentenabgabevorrichtung eine Insulinabgabevorrichtung ist, die zum Abgeben von Insulin an den Benutzer gestaltet ist.

## Revendications

1. Instrument permettant de percer la peau d'un utilisateur et de transporter un médicament vers l'utilisateur, comprenant :
un corps cylindrique ayant une lumière interne pour transporter le médicament ;
un élément perforant formé en tant que pointe perforante pointue en polymère soluble ou dégradable et fixé à une extrémité distale du corps cylindrique pour percer la peau de l'utilisateur et positionner l'instrument au moins partiellement sous la peau de l'utilisateur ; et dans lequel le corps cylindrique est conçu pour se ramollir, et l'élément perforant est conçu pour se dissoudre ou se dégrader, y compris se séparer de l'instrument (300) par rupture lorsqu'il est positionné au moins partiellement sous la peau de l'utilisateur,
**caractérisé en ce que**
le corps cylindrique est formé d'un matériau qui est rigide avant exposition aux liquides corporels et/ou à la chaleur corporelle, mais qui ramollit lorsque le matériau se trouve sous la peau de l'utilisateur.

2. Instrument selon la revendication 1, dans lequel le matériau du corps cylindrique est le polytétrafluoroéthylène (PTFE), le polyéthylène haute densité (PEHD), un polysaccharide, un polyhydroxyalcanoate (PHA) ou un acide polylactique.

3. Instrument selon la revendication 1 ou 2, dans lequel le matériau du corps cylindrique ramollit jusqu'à un état semi-rigide lorsqu'il est positionné sous la peau d'un utilisateur.

4. Instrument selon l'une quelconque des revendications 2 à 3, dans lequel le matériau de l'élément perforant comprend ou consiste en un polymère soluble ou dégradable, tel que l'alcool polyvinylique (PVA), un polymère à base d'amidon ou un polymère biodégradable, tel que l'acide polylactique-co-glycolique (PLGA) ou l'acide polyglycolique (PGA).

5. Instrument permettant de percer la peau d'un utilisateur et de transporter un médicament vers l'utilisateur, comprenant :
un corps cylindrique formé d'un matériau mou et présentant une lumière interne pour transporter le médicament ;
des éléments activables qui sont noyés ou moulés dans le matériau souple et **caractérisés en ce que** les éléments activables sont activables pour amener l'instrument à se rigidifier afin de faciliter le perçage de la peau de l'utilisateur,
dans lequel les éléments activables sont organisés par paires réparties régulièrement sur la circonférence et la longueur de l'extérieur de l'instrument,
dans lequel les éléments activables se présentent sous la forme de fils en alliage à mémoire de forme (AMF) qui se rigidifient lorsque de l'électricité est appliquée aux fils AMF, et ainsi rigidifient l'instrument.

6. Instrument selon la revendication 5, dans lequel le ou les fils AMF sont enroulés hélicoïdalement sur une longueur du corps cylindrique.

7. Dispositif d'administration de médicament, comprenant :
un réservoir pour stocker un médicament ;
l'instrument selon l'une quelconque des revendications précédentes, dans lequel l'instrument est conçu pour rester au moins partiellement positionné sous la peau de l'utilisateur pour transporter le médicament vers l'utilisateur ; et
un chemin de fluide reliant le réservoir à l'instrument de sorte que le médicament puisse passer du réservoir à l'instrument.

8. Dispositif d'administration de médicament selon la revendication 7, comprenant en outre :
un composant coulissant pour faire coulisser l'instrument vers une première position pour percer la peau de l'utilisateur et positionner l'instrument sous la peau de l'utilisateur et pour faire coulisser l'instrument à l'écart de l'utilisateur dans une seconde position où l'instrument est rétracté de l'utilisateur ;
et un rail, un ressort et une liaison reliés au ressort et au composant coulissant pour entraîner le composant coulissant unique.

9. Dispositif d'administration de médicament selon l'une quelconque des revendications 7 à 8, dans lequel le dispositif d'administration de médicament est un dispositif d'administration d'insuline conçu pour administrer de l'insuline à l'utilisateur.
